# EUROPÄISCHE PATENTSCHRIFT

(11) **EP 2 475 992 B1**
(45) Veröffentlichungstag und Bekanntmachung des Hinweises auf die Patenterteilung: **06.01.2016**
(21) Anmeldenummer: 10752355.7
(22) Anmeldetag: 13.09.2010
(51) Int. Cl.: G01N 33/50

(54) **VERFAHREN ZUR DIAGNOSE UND/ODER VORHERSAGE DER ENTWICKLUNG VON NEURODEGENERATIVEN ERKRANKUNGEN**
METHOD FOR DIAGNOSING AND/OR PREDICTING THE DEVELOPMENT OF NEURODEGENERATIVE DISEASES
PROCÉDÉ POUR LE DIAGNOSTIC ET/OU LE PRONOSTIC DU DÉVELOPPEMENT DE MALADIES NEURODÉGÉNÉRATIVES

(30) Priorität: 11.09.2009 DE 102009042160
(43) Veröffentlichungstag der Anmeldung: 18.07.2012
(73) Patentinhaber: Eberhard-Karls-Universität Tübingen Universitätsklinikum, 72076 Tübingen (DE)
(72) Erfinder: BISKUP, Saskia, 70599 Stuttgart (DE); FUNK, Natalja, 72072 Tuebingen (DE)
(74) Vertreter: Witte, Weller & Partner Patentanwälte mbB
(86) Internationale Anmeldenummer: PCT/EP2010/063406
(87) Internationale Veröffentlichungsnummer: WO 2011/029939

(56) Entgegenhaltungen:
- WO-A1-2008/003516
- CHOI CHUL WON ET AL: "Impaired differentiation and apoptosis of hematopoietic precursors in a mouse model of myelodysplastic syndrome", HAEMATOLOGICA-THE HEMATOLOGY JOURNAL, Bd. 93, Nr. 9, September 2008 (2008-09), Seiten 1394-1397 URL, XP002610856, ISSN: 0390-6078
- LEE S -T ET AL: "Reduced circulating angiogenic cells in Alzheimer disease", NEUROLOGY, Bd. 72, Nr. 21, Mai 2009 (2009-05), Seiten 1858-1863, XP002610857, ISSN: 0028-3878
- BJORKQVIST MARIA ET AL: "A novel pathogenic pathway of immune activation detectable before clinical onset in Huntington's disease", JOURNAL OF EXPERIMENTAL MEDICINE, Bd. 205, Nr. 8, August 2008 (2008-08), Seiten 1869-1877, XP002610858, ISSN: 0022-1007

## Beschreibung

Die vorliegende Erfindung betrifft ein Verfahren zur Diagnose und/oder Vorhersage der Entwicklung von neurodegenerativen Erkrankungen.

Neurodegenerative Erkrankungen bzw. Krankheiten zeichnen sich durch einen langsamen, unaufhaltsamen Nervenzelltod aus. Zu den neurodegenerativen Krankheiten des Menschen zählen u.a. die amyotrophe Lateralsklerose (ALS), Tauopathien, wie z.B. die Alzheimersche Krankheit, Trinucleotidkrankheiten, wie z.B. Chorea Huntington (Chorea), Prionen-Krankheiten, wie z.B. die Creutzfeldt-Jakob-Erkrankung, und -Synucleinopathien, wie z.B. die Parkinsonsche Krankheit. Insbesondere die Alzheimer Krankheit und die Parkinson'sche Krankheit sind dabei eine häufige Ursache von Demenz und hieraus resultierender Pflegebedürftigkeit im Alter.

Bisher sind auf dem Markt keine Test bekannt, mit Hilfe derer neurodegenerative Erkrankungen, insbesondere solche, die nicht genetisch bedingt sind, frühzeitig und zweifelsfrei festgestellt werden können. Eine frühzeitige Erkennung von Personen, die das Risiko tragen, eine neurodegenerative Erkrankung zu entwickeln, wäre aber wünschenswert, da bei einer erst spät diagnostizierten neurodegenerativen Erkrankung ein Großteil der Nervenzellen in der betroffenen Person/im Patienten bereits degeneriert ist, und eine Therapie in Folge dessen erst zu spät einsetzt. Mit einer frühzeitigen Identifizierung von Risikopatienten und einer frühzeitig einsetzenden Therapie könnte der Verlust von Nervenzellen dieser Patienten verhindert, zumindest aber frühzeitig gebremst werden.

In der DE 10 2007 024 382 A1 ist ein Verfahren zur Diagnose einer neurodegenerativen Erkrankung beschrieben, bei dem die Stärke der Genexpression bestimmter Gene in einer Biopsie-Probe eines Patienten untersucht wird. Dieses Verfahren hat den Nachteil, dass die Biopsie-Entnahme für den untersuchenden Patienten unangenehm und schmerzhaft sein kann, und dass eine zuverlässige Aussage über das Risiko, eine neurodegenerative Erkrankung zu entwickeln. Darüber hinaus ist der Material- und damit auch der Kostenaufwand groß, da für eine zuverlässige Diagnose in der Regel mehrere Gene nachgewiesen werden müssen.

Ferner sind im Stand der Technik verschiedene Mutationen des LRKK2-Gens bekannt, die als genetische Marker für die familiäre Parkinson'sche Erkrankung gehandelt werden. Mit diesen Markern kann jedoch lediglich das Risiko zur Entwicklung einer spezifischen Erkrankung bestimmt werden, wobei die Marker darüber hinaus auch nur familiäre Marker darstellen.

Lee et al. ("Reduced circulating angiogenic cells in Alzheimer disease", (2009), Neurology 72: 1858 - 1863) beschreiben die Erkenntnis, dass bei Alzheimer-Erkrankten weniger angiogene Zellen im Blut zirkulieren als bei gesunden Vergleichspatienten. Diese Erkenntnisse führten die Autoren dieser Veröffentlichung darauf zurück, dass bei Alzheimer-Erkrankten eine geringere Anzahl an CFU-EPC zu finden waren als bei gesunden Kontroll-Patienten.

Choi et al. ("Impaired differentiation and apoptosis of hematopoietic precursors in a mouse model of myelodyplastic syndrome", (2008), Haematologica 93: 1394 - 1397) offenbaren den Einsatz eines CFC-Assays, bei welchem ein Methylcellulose-Medium eingesetzt wird.

Daher liegt der vorliegenden Erfindung die Aufgabe zugrunde, ein neues Verfahren bzw. neue Marker bereit zu stellen, mit denen die Nachteile des Standes der Technik überwunden und das Risiko zur Entwicklung von neurodegenerativen Krankheiten schnell, gut verträglich für die zu untersuchenden Person und zuverlässig vorhergesagt werden kann.

Erfindungsgemäß wird diese Aufgabe gelöst durch ein Verfahren zur Diagnose und/oder Vorhersage der Entwicklung von neurodegenerativen Erkrankungen, wobei das Verfahren die in den Ansprüchen aufgeführten Schritte aufweist.

Die Erfinder haben bei der Entwicklung des erfindungsgemäßen Verfahrens den Umstand berücksichtigt, dass über das Blut und das lymphatische System ein ständiger Austausch zwischen den Nervenzellen des Gehirns auf der einen und den Zellen des Immunsystems auf der anderen Seite besteht. Neurodegenerative Erkrankungen führen unter anderem zum Tod von einzelnen Nervenzellen. Die Ursachen hierfür können vielseitig sein. Untergehende Nervenzellen wiederum führen zur Freisetzung von Signalstoffen, die Gehirn-residente Makrophagen sowie weitere Zellen des Immunsystems anlocken. Das Einwandern von Immunzellen wiederum bedingt einen Nervenzelluntergang, so dass es zu einer nicht mehr kontrollierbaren Reaktion kommt, die letztendlich die Grundlage für die langsam fortschreitende neurodegenerative Erkrankung bildet. Darüber hinaus werden Signalstoffe in das Blut und das lymphatische System abgegeben. Diese Signalstoffe entfalten auch eine Wirkung auf weiße Blutkörperchen in der Peripherie sowie auf deren Vorläuferzellen beispielsweise im Knochenmark. Dies kann unter anderem die Proliferation und Differenzierung bestimmter weißer Blutkörperchen initiieren. Diesen Anstieg an weißen Vorläuferzellen macht sich das erfindungsgemäße Verfahren zunutze.

Das erfindungsgemäße Verfahren weist dabei die folgenden Schritte auf:,
a) Isolieren von weißen Blutkörperchen aus einer Blutprobe einer zu untersuchenden Person;
b) Anreicherung und/oder Kultivierung der in Schritt a) isolierten weißen Blutkörperchen, zum Beispiel in einem Medium, zur Bildung von verschiedenen Kolonie-bildenden Einheiten (CFUs); und
c) Bestimmen und Auswerten der relativen Anzahl der in Schritt b) gebildeten CFU-M im Verhältnis zu den anderen gebildeten CFUs.

Ferner wird die der Erfindung zugrunde liegende Aufgabe gelöst durch die Verwendung eines CFC Assays und eines Methylcellulose enthaltenden Mediums, in dem weiße Blutkörperchen unter Bildung verschiedener Kolonien kultiviert werden können, zur Diagnose und/oder Vorhersage der Entwicklung von neurodegenerativen Erkrankungen.

Schließlich wird die der Erfindung zugrunde liegende Aufgabe durch ein Kit gelöst, das ein Methylcellulose enthaltendes Medium sowie eine Anleitung zur Durchführung des erfindungsgemäßen Verfahrens enthält.

Die der Erfindung zugrunde liegende Aufgabe wird auf diese Weise vollkommen gelöst.

Mit dem neuen Verfahren, bzw. der neuen Verwendung und dem neuen Kit, ist es nun erstmals möglich, durch Untersuchung einer Blutprobe, bzw. durch Untersuchung der Kolonie-Bildung der hieraus isolierten weißen Blutkörperchen, zu bestimmen, ob die Person, von der die zu untersuchende Blutprobe stammt, das Risiko trägt, eine neurodegenerative Erkrankung zu entwickeln oder nicht. Mit dem erfindungsgemäßen Verfahren wird nämlich untersucht, welchen Anteil die CFU-M an der Gesamtzahl der nach der Kultivierung der weißen Blutkörperchen erhaltenen Kolonien haben. Die Erfinder der vorliegenden Anmeldung haben einerseits gezeigt, dass bei Parkinson-Patienten der Anteil an gebildeten CFU-M größer ist als bei gesunden Kontrollpersonen. Ferner wurde mittels des erfindungsgemäßen Verfahrens gezeigt, dass auch bei Personen, die Mutationen in einem bestimmten Gen tragen, das als Marker für familiäre Parkinson'sche Erkrankung gilt, der Anteil an gebildeten CFU-M größer ist als bei Kontroll-Personen. Daher lässt das Vorliegen eines höheren CFU-M-Kolonieanteils auf das Risiko einer Person schließen, eine neurodegenerative Erkrankung zu entwickeln.

Unter "CFU" und "CFC" wird wie im Stand der Technik Folgendes verstanden: CFC ("colony-forming cells"; "Kolonie-bildende Zellen") stellen Stammzellen/Vorläuferzellen dar, die im Gegensatz zu pluripotenten Stammzellen weiter differenziert sind und auf bestimmte Zelldifferenzierungs-Linien festgelegt sind. Im Koloniebildungstest (colony-forming unit-culture (CFU-C) assay) können sie durch die Gabe von koloniestimulierenden Faktoren (CSF) zur Bildung von Kolonien dieser Zelllinie angeregt werden. Die Zellen der entstehenden Kolonien können dann aufgrund ihrer Morphologie und bestimmter Oberflächenmarker identifiziert werden, und die Vorstufen werden danach in sog. CFU (colony forming units) eingeteilt: So unterscheidet man - und vorliegend fallen unter den Begriff "CFU" die folgenden - bspw. CFU-Bas ("basophil"; Vorläuferzelle blutbildender Zellen mit Erkennungsmarker CD34); CFU-E ("erythrocyte"; Vorläuferzelle von Erythrocyten); CFU-Eo (Eo-CFC, "eosinophil"; Vorstufe von eosinophilen Granulocyten); CFU-G (G-CFC, "granulocyte"; Vorläuferzelle von Granulocyten); CFU-GEMM ("granulocyte, erythrocyte, megakaryocyte, macrophage"; Vorläuferzelle von Granulozyten, Erythrozyten, Megakaryozyten, Makrophagen); CFU-GM (GM-CFC, "granulocyte, macrophage"; Vorläuferzelle von Granulozyten, Makrophagen); CFU-M (M-CFC, "macrophage"; Vorläuferzelle von Makrophagen); CFU-MEG ("megakaryocyte"; Vorläuferzelle von Megakaryocyten).

Hämatopoetische Stamm- und Vorläuferzellen finden sich nicht nur u.a. im Knochenmark, sondern auch im Peripherblut. Durch die Isolierung von weißen Blutkörperchen aus dem Peripherblut werden daher auch Vorläuferzellen isoliert, die unter bestimmten Kultivierungsbedingungen proliferieren und differenzieren und die o.g. Kolonien bilden. Anders gesprochen können daher über die Koloniebildung im CFC Assay hämatopoetische Vorläuferzellen in einer Probe ausgezählt werden.

Bei dem erfindungsgemäßen Verfahren werden also zunächst die weißen Blutzellen aus einer bereitgestellten Blutprobe, die vorteilhafterweise frisches Blut (heparinisiertes Peripherblut) enthält, von den anderen Blutbestandteilen isoliert, bspw. durch Gradientenzentrifugation oder über Antikörper und in einem Medium, das die Kolonie-Bildung zulässt, kultiviert. Die Kultivierungsdauer beträgt dabei mindestens 10 Tage, vorzugsweise 14 bis 20 Tage. Nach der Kultivierung werden die gebildeten Kolonien ausgezählt und die Werte der bestimmten einzelnen CFUs verglichen.

Die Erfinder haben nun in eigenen Versuchen gezeigt, dass sowohl Patienten, die an Parkinson leiden, als auch Personen, die die oben erwähnte Mutation in dem LRRK2-Gen ("Leucin-rich repeat Kinase 2"- Gen, Leucin reiche Kinase 2-Gen) tragen (aber noch keine Anzeichen von Parkinson zeigten), Werte hinsichtlich der CFU-Bildung zeigten, die sich von denen von Kontrollproben unterschieden. In einer Ausführungsform des erfindungsgemäßen Verfahrens ist daher bevorzugt, wenn in Schritt c) die relative Anzahl von zumindest zwei der folgenden in Schritt b) gebildeten CFUs bestimmt und verglichen wird, nämlich CFU-G (CFU-Granulozyt) und CFU-M (CFU-Makrophage). In einer weiteren Ausführungsform wird zusätzlich noch die relative Anzahl der CFU-GM (CFU-Granulozyt/Makrophage) bestimmt.

Durch den Vergleich oder Abgleich der Werte für die relative Anzahl der verschiedenen CFUs kann bestimmt werden, ob der Wert für die CFU-M höher liegt als bspw. für die CFU-G.

Insgesamt zeigte sich, dass bei Parkinson-Patienten, bzw. bei Personen mit Risiko zur Entwicklung von Parkinson, die relative Anzahl von CFU-M höher war als bei den Kontrollproben; daher ist in einer Ausführungsform des erfindungsgemäßen Verfahrens bevorzugt, wenn jeweils die relative Anzahl von CFU-M und CFU-G bestimmt und diese Werte bei Kranken und Gesunden verglichen werden. Ein erhöhter Wert für die CFU-M wird dabei mit dem Risiko assoziiert, neurodegenerative Erkrankungen zu entwickeln.

Dabei bedeutet vorliegend "relative Anzahl" der Anteil einer bestimmten Kolonieform im Verhältnis zur Gesamtzahl der gebildeten Kolonien.

Die Blutprobe ist ferner vorzugsweise frisch, und wurde zuvor einer Person entnommen, der hinsichtlich des Risikos, neurodegenerativen Erkrankung zu entwickeln, untersucht werden soll. Es versteht sich dabei, dass die Person oder der Patient ein Mensch ist, wobei das Geschlecht und Alter sowie körperliche Verfassung keine Rolle spielen insofern keine Erkrankungen vorliegen (Erkältung, Infektion etc).

Vorliegend - wie auch im betreffenden Fachgebiet selber - werden unter weißen Blutzellen oder weißen Blutkörperchen (Leukozyten) die kernhaltige Blutzellen mit Abwehrfunktion verstanden, und umfassen Granulocyten, Lymphocyten und Monocyten.

Bei einer Ausführungsform des erfindungsgemäßen Verfahrens wird daher in Schritt c) eine bestimmte relative Anzahl von CFU-M mit dem Vorliegen und/oder dem Verlauf und/oder der Stärke und/oder der Vorhersage von neurodegenerativen Erkrankungen assoziiert.

Insbesondere ist dabei bevorzugt, wenn eine CFU-M-Kolonienanzahl von >25 % - berechnet auf die Gesamtzahl der Kolonien - mit dem Vorliegen und/oder Verlauf und/oder der Stärke und/oder der Vorhersage von neurodegenerativen Erkrankungen assoziiert wird.

Dabei versteht sich, dass im Rahmen der vorliegenden Erfindung auch Werte in Betracht kommen, die etwas unterhalb von 25 % liegen, aber noch im Rahmen des Verständnisses des Fachmanns bei Lesen der Erfindung und unter Berücksichtigung von ev. Auszählfehlern als noch im Rahmen der Erfindung liegen.

Dabei ist in einer Ausführungsform des erfindungsgemäßen Verfahrens bevorzugt, wenn das in Schritt b) verwendete Medium zum Kultivieren Methylcellulose oder andere gallertartige Substanzen enthält.

Durch Methylcellulose oder andere gallertartige Substanzen, bspw. Agarose, bildet sich eine halbfeste Matrix, in der die Zellen kultiviert werden. Vorteilhafterweise enthält das Kultivierungsmedium Methylcellulose, es versteht sich, dass aber auch jeder andere Stoff, der zur Bildung einer halbfesten Matrix und zur Kultivierung von Zellen darin geeignet ist, eingesetzt werden kann.

Die Erfindung betrifft ferner die Verwendung eines CFC Assays zur Diagnose und/oder Vorhersage der Entwicklung von neurodegenerativen Erkrankungen.

Unter CFC Assay (colony forming cell (CFC) assay), das auch als Methylcellulose Assay bezeichnet wird, wird vorliegend, wie auch im Stand der Technik ein *in vitro* Assay verstanden, das auf der Fähigkeit der hämatopoetischen Vorläuferzellen/Vorstufen basiert, in einem halbfesten Medium (mit Cytokin-Stimulation) in Kolonien zu proliferieren und zu differenzieren. Die gebildeten Kolonien können dann hinsichtlich ihrer Morphologie ausgezählt werden. Erfindungsgemäß können also die an sich im Stand der Technik bekannten und erhältlichen CFC Assays für die Diagnose und/oder die Vorhersage der Entwicklung von neurodegenerativen Erkrankungen eingesetzt werden.

Die Erfinder haben vorliegend erstmalig gezeigt, dass über die Verwendung von Methylcellulose enthaltendem Medium sowie dem mit diesem Medium anzuwendenden CFC Assay ein Mittel bereitgestellt wird, mit dem sich über die Anwendung des erfindungsgemäßen Verfahrens das Risiko zur Entwicklung von neurodegenerativen Erkrankungen eines Menschen ermitteln lässt.

Es versteht sich, dass die obenstehend beschriebenen und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird in der nachfolgenden Beschreibung der Beispiele bzw. Ausführungsbeispiele sowie anhand der Figuren näher erläutert. Es zeigen:
- Fig. 1: eine schematische Übersicht der Hämatopoese;
- Fig. 2: eine Übersicht über die Verteilung der im CFC Assay (Kulturmedium mit Erythropoietin (Epo)) gebildeten verschiedenen Kolonien aus Proben von Kontrollpersonen; Säulendiagramm (A) und mikroskopische Morphologische der jeweiligen Klone (B);
- Fig. 3: die Verteilung der im CFC Assay (Kulturmedium ohne Epo) gebildeten Klone in Proben von Kontrollpersonen (A) und in Proben von Parkinson-Patienten (B), jeweils dargestellt im Säulendiagramm;
- Fig. 4: ein Diagramm, das die relative Anzahl (in %) von CFU-M verschiedener menschlicher Populationen, darunter Träger von LRRK2-Mutationen und Parkinson-Patienten, wiedergibt;

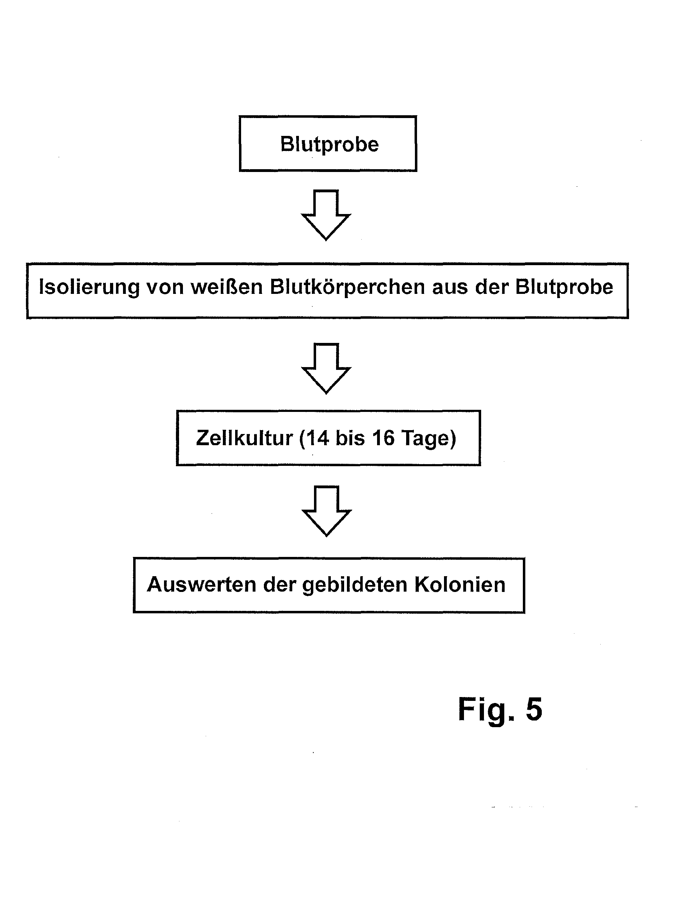

- Fig. 5: eine schematische Übersicht über die Schritte einer Ausführungsform des erfindungsgemäßen Verfahrens.

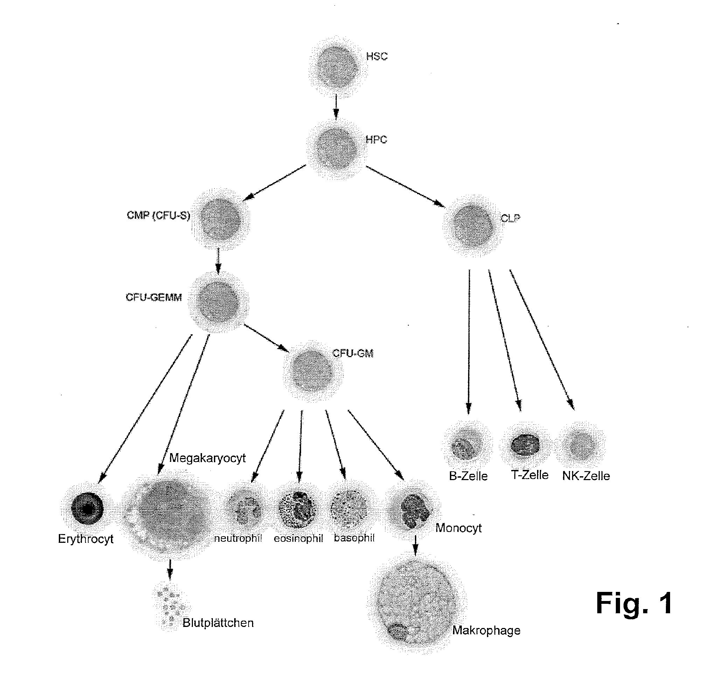

In Fig. 1 ist schematisch eine Übersicht über die Hämatopoese im Menschen gezeigt, wobei die in Fig. 1 aufgeführten Abkürzungen Folgendes bedeuten: HSC: hämatopoetische Stammzellen, HPC: hämatopoetische Vorläuferzellen; CMP (CFU-S): "common myeloid precursor", gemeinsamer myeloider Vorläufer; CLP: "common lymphoid precursor", gemeinsamer lymphoider Vorläufer; CFU-GEMM: "Colony forming unit granulocyte erythroid megakaryocyte macrophage", gemischte Kolonien; CFU-GM: "colony folrming unit granulocyte macrophage", Granulozyt-Makrophagen-Koloniebildende Einheit. Die Hämatopoese ist ein zellulärer Teilungs- und Reifungsvorgang, der die Blutzellen hervorbringt. Ausgang der Blutbildung ist die pluripotente, undifferenzierte hämatopoetische Stammzelle (siehe in Fig. 1: HSC), die Vorstufen (oder Vorläuferzellen) hervorbringt, die sich nicht selbst erneuern können und nur einen spezialisierten Zelltyp zur Ausreifung bringen. Die unreifen Vorläuferzellen könen im Blut zirkulieren und sich wieder im Knochenmark ansiedeln. Die Regulation der Blutbildung erfolgt durch Milieufaktoren oder humoral (z. B. durch Cytokine, Hormone, Chalone, Erythropoetin). Weiter ausgehend vom gemeinsamen myeloiden Vorläufer CMP bilden sich - über die Zwischenstufe CFU-GEMM - neutrophile, eosinophile und basophile Granulocyten (über die weitere Vorstufe CFU-GM), sowie Erythrocyten, Megakaryocyten und Monocyten.

### Beispiel

### Untersuchung der Klon-Verteilung bei Kontrollproben und bei Kultivierung mit EPO

In Ausgangsversuchen wurden Proben von Kontrollpersonen unterschiedlichen Geschlechts und Alters hinsichtlich der Verteilung der Klone nach Kultivierung der weißen Blutzellen aus diesen Proben in einem Medium mit Erythropoetin untersucht. Hierzu werden die weissen Blutzellen nach Gradientenzentrifugation gewonnen, gewaschen, ausgezählt und in ein Methylcellulose und Zytokine (Epo (Erythropoetin), SCF (Stammzellfaktor; stem cell factor), GM-CSF (Granulocyten-Makrophage-Kolonie-stimulierender Faktor), IL-3 (Interleukin-3) (alle R & D Systems, Minneapolis, USA) enthaltendes Medium aufgenommen.

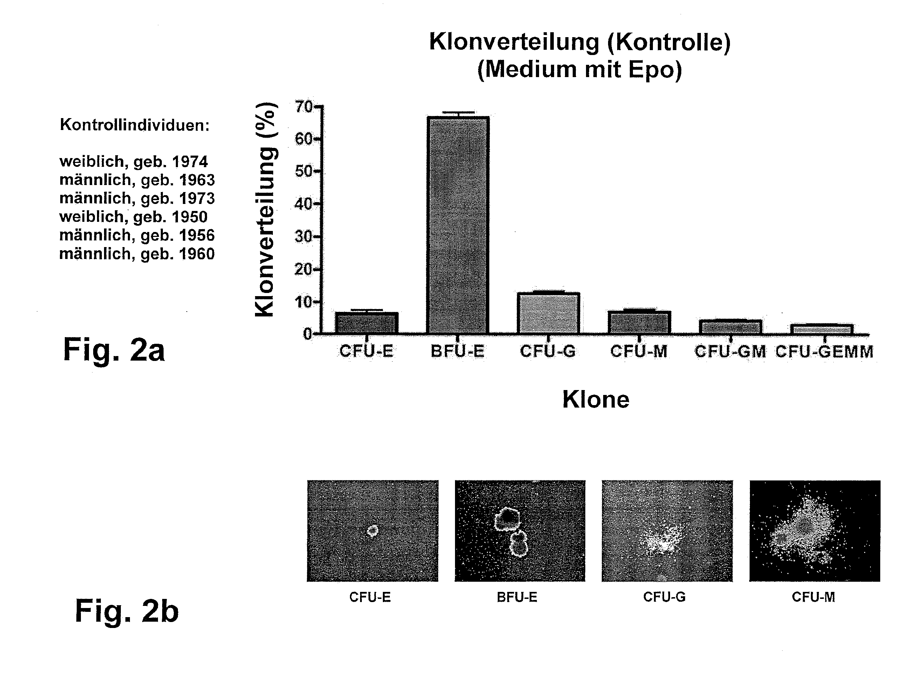

Nach 14-tägiger Inkubation zeigte sich die in Fig. 2 wiedergegebene Verteilung: In Fig. 2A sind die Ergebnisse in einem Säulendiagramm wiedergegeben, und die korrespondierenden Aufnahmen der jeweiligen Klone in den Fotografien unterhalb des Säulendiagramms (Fig. 2B), unterhalb des jeweiligen Klons. Es zeigte sich, dass die prozentualen Anteile der verschiedenen Vorläuferzellen (CFU-E, BFU-E, CFU-G, CFU-M, CFU-GM, CFU-GEMM) bei Kontrollpersonen unabhängig von Geschlecht und Alter sich kaum unterscheiden.

### Untersuchung der Klon-Verteilung bei Kontrollproben und bei Kultivierung ohne EPO

In anschließenden Versuchen wurden Proben von gesunden Kontrollpersonen und Proben von Patienten mit Parkinson'scher Erkrankung untersucht. Hierzu wurden jeweils 10 ml heparinisiertes Peripherblut der Personen/Patienten jeweils durch Ficoll-Dichte-Gradientenzentrifugation aufgetrennt, und mit den daraus separierten weißen Blutzellen eine Zellkultur angelegt. Hierzu wurde ein kommerziell erhältliches Medium, das Methylcellulose und weitere Zusatzstoffe (SCF, GM-CSF, IL-3) enthielt, eingesetzt (R & D Systems, Minneapolis, USA). Die Zellkultur wurde 14 Tage in einem Brutschrank kultiviert, die die gebildeten Zellen bzw. Kolonien anschließend ausgezählt.

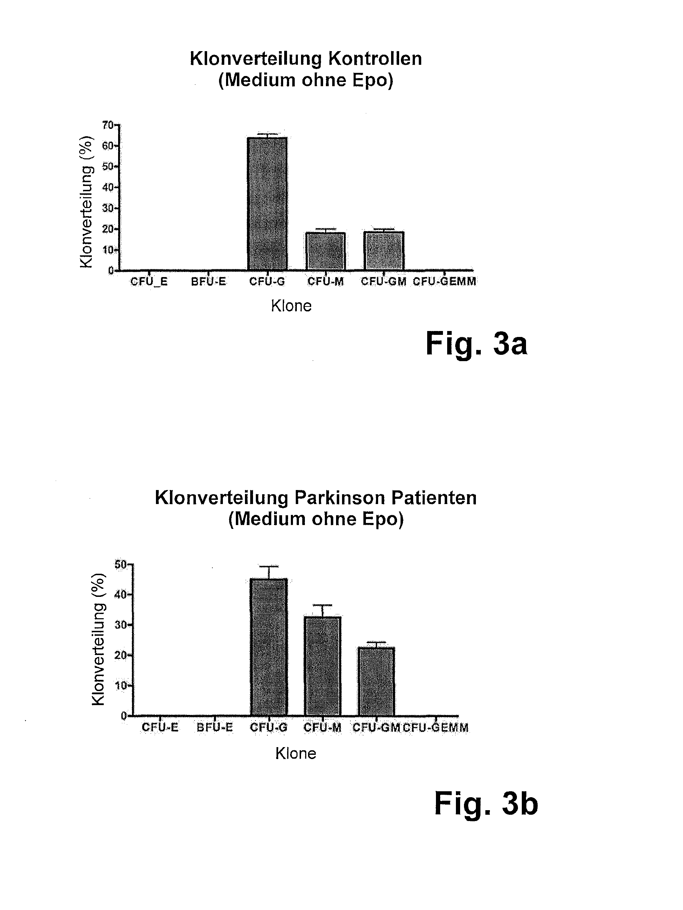

Es zeigte sich, dass ausgehend von den Proben der Kontrollpersonen ca. 63% CFU-G, ca. 18 % CFU-M und ca. 19 % CFU-GM gebildet wurden (siehe Fig. 3A). Bei den Proben der an Parkinson erkrankten Personen (siehe Fig. 3B) zeigte sich hingegen eine Verteilung von ca. 45 % CFU-G, ca. 33 % CFU-M und ca. 22 % CFU-GM, und zeigt somit eine deutliche Verschiebung der Kolonie-Bildung in Richtung CFU-M.

### Untersuchung von Proben von Personen mit LRRK2-Mutation

In weiteren Versuchen wurden Proben von Personen untersucht, die Träger verschiedener Mutationen im LRRK2-Gen sind. Mutationen im LRRK2-Gen ("Leucin-rich repeat Kinase 2"- Gen, Leucin reiche Kinase 2-Gen) sind nach neueren Erkenntnissen Biomarker für die familiäre Parkinson'sche Erkrankung; bisher identifiziert wurden die LRRK2-Mutationen G20192, Q930R, und L1114L.

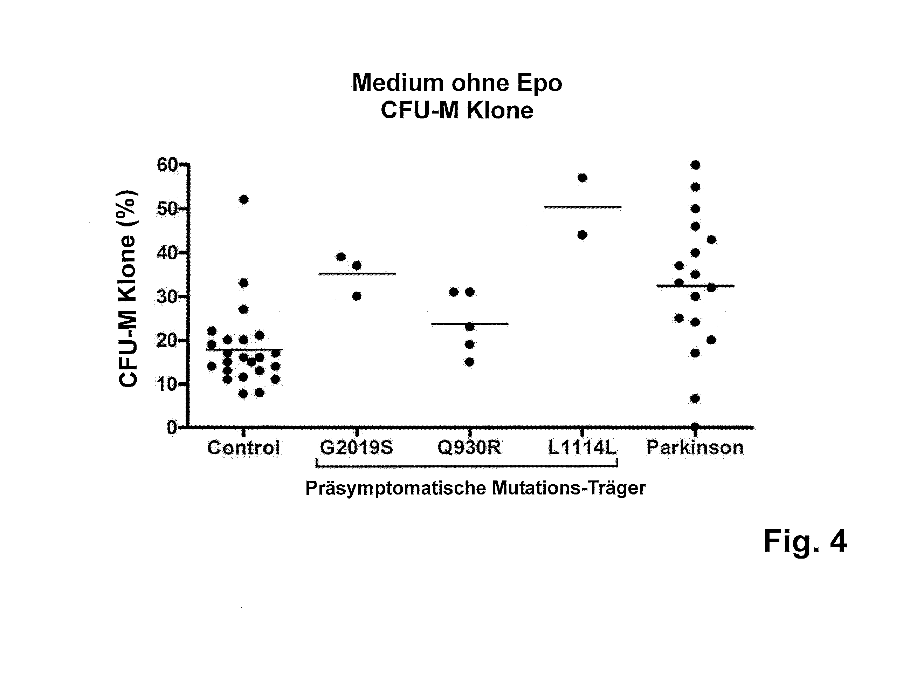

Die Versuchsdurchführung erfolgte wie oben beschrieben. Die Kolonie-Bildung von Proben von Personen mit diesen Mutationen wurden mit Kontrollen sowie mit derjenigen von Parkinson-Patienten verglichen (siehe Fig. 4). Hierbei bestätigten sich die bereits für die Parkinson-Patienten getroffenen Erkenntnisse, nämlich dass die relative Anzahl der CFU-M bei Trägern der Mutationen und bei Parkinson-Erkrankten durchschnittlich höher war als bei den Kontrollen.

Damit konnten die Erfinder zeigen, dass das Verfahren ein geeignetes Mittel darstellt, über welches Aussagen über das Risiko zur Entwicklung von neurodegenerativen Erkrankungen gemacht werden können.

Die Blutproben wurden im Rahmen einer wissenschaftlichen Studie der Abteilung fuer Neurodegeneration am Hertie Institut fuer klinische Hirnforschung, Tübingen, gewonnen. Ein Ethikantrag lag vor.

Das Blut (ca. 10 ml) wurde 1:1 mit HBSS (Hank's Balanced Salt Solution, Invitrogen, Carlsbad, USA) verdünnt und über ein 15 ml Ficoll-Paque Plus (GE Healthcare) geschichtet. Durch Zentrifugation für 30min bei 400 x g werden weiße Blutkörperchen gewonnen, diese 2x mit je 20 ml HBSS gewaschen, in 10 ml IMDM (Iscove's Modified Dulbecco's Media, Invitrogen) aufgenommen und ausgezählt. Zur weiteren Kultivierung der gewonnenen weißen Blutkörperchen wurden Methylcellulose enthaltene Medien von R&D Systems verwendet (Human Methylcellulose Complete Media und Human Methylcellulose Complete Media ohne Epo). Es wurden ca. 500.000 Zellen /ml Medium ausplattiert. Die Kultivierung der Zellen erfolgte in 3,5 cm Zellkulturschalen (BD Biosciences Falcon, San Jose, USA) bei 37°C, 5% CO₂ und hoher Luftfeuchtigkeit. Am Tag 15 nach dem Ausplattieren wurden die Kolonien lichtmikroskopisch ausgewertet und die prozentuellen Anteile der einzelnen Klonpopulationen berechnet.

## Patentansprüche

1. Verfahren zur Diagnose und/oder Vorhersage der Entwicklung von neurodegenerativen Erkrankungen, wobei das Verfahren die folgenden Schritte aufweist:
a) Isolierung von weißen Blutzellen aus einer Blutprobe einer zu untersuchenden Person;
b) Anreicherung und/oder Kultivierung der in Schritt a) isolierten weißen Blutzellen zur Bildung von verschiedenen Kolonie-bildenden Einheiten (CFUs), wobei sich CFU-M und weitere CFU bilden, und
c) Bestimmen und Auswerten der relativen Anzahl der in Schritt b) gebildeten CFU-M im Verhältnis zu den anderen gebildeten CFUs.

2. Verfahren nach Anspruch 1, **dadurch gekennzeichnet, dass** Schritt b) in einem Medium zur Bildung von verschiedenen Kolonie-bildenden Einheiten (CFUs) durchgeführt wird.

3. Verfahren nach Anspruch 1 oder 2, **dadurch gekennzeichnet, dass** in Schritt c) die relative Anzahl von zumindest zwei der folgenden in Schritt b) gebildeten CFUs bestimmt und verglichen wird, nämlich CFU-G (CFU-Granulozyt) und CFU-M (CFU-Makrophage).

4. Verfahren nach Anspruch 3, **dadurch gekennzeichnet, dass** ferner die relative Anzahl von CFU-GM (CFU-Granulozyt/Makrophage) bestimmt wird.

5. Verfahren nach einem der Ansprüche 1 bis 4, **dadurch gekennzeichnet, dass** das Bestimmen einer relativen Anzahl von CFU-M, die höher ist als die relative Anzahl von CFU-G, mit dem Vorliegen und/oder dem Verlauf und/oder der Stärke und/oder der Vorhersage von neurodegenerativen Erkrankungen assoziiert wird.

6. Verfahren nach einem der Ansprüche 1 bis 4, **dadurch gekennzeichnet, dass** in Schritt c) eine bestimmte relative Anzahl von CFU-M mit dem Vorliegen und/oder dem Verlauf und/oder der Stärke und/oder der Vorhersage von neurodegenerativen Erkrankungen assoziiert wird.

7. Verfahren nach Anspruch 6, **dadurch gekennzeichnet, dass** eine CFU-M-Kolonienanzahl von mindestens ca. 25 % mit dem Vorliegen und/oder Verlauf und/oder der Stärke und/oder der Vorhersage von neurodegenerativen Erkrankungen assoziiert wird.

8. Verfahren nach einem der Ansprüche 1 bis 7, **dadurch gekennzeichnet, dass** das in Schritt b) verwendete Medium zum Kultivieren Methylcellulose enthält.

9. Verfahren nach einem der Ansprüche 1 bis 8, **dadurch gekennzeichnet, dass** das Isolieren der weißen Blutzellen aus der Blutprobe über eine Dichte-Gradientenzentrifugation oder Aufreinigung mittels spezifischer Antikörper erfolgt.

10. Verfahren nach einem der Ansprüche 1 bis 9, **dadurch gekennzeichnet, dass** das Kultivieren der isolierten weißen Blutzellen über einen Zeitraum von mindestens 10 Tagen, vorzugsweise 14 Tagen erfolgt.

11. Verwendung eines CFC-Assays mit Koloniebildung hämatopoetischer Vorläuferzellen in einer Probe zur Diagnose und/oder Vorhersage der Entwicklung von neurodegenerativen Erkrankungen.

## Claims

1. A method for diagnosing and/or predicting the development of neurodegenerative diseases, wherein the method comprises the following steps:
a) isolation of white blood cells from a blood sample from a person to be assessed;
b) enrichment and/or cultivation of the white blood cells isolated in step a) for forming various colony-forming units (CFUs), wherein CFU-M and other CFUs are formed, and
c) determination and evaluation of the relative number of CFU-M formed in step b) relative to the other CFUs formed.

2. The method as claimed in claim 1, **characterized in that** step b) is carried out in a medium for forming various colony-forming units (CFUs).

3. The method as claimed in claim 1 or 2, **characterized in that** in step c) the relative number of at least two of the following CFUs formed in step b) is determined and compared, namely CFU-G (CFU-granulocyte) and CFU-M (CFU-macrophage).

4. The method as claimed in claim 3, **characterized in that** additionally the relative number of CFU-GM (CFU-granulocyte/macrophage) is determined.

5. The method as claimed in any of claims 1 to 4, **characterized in that** the determination of a relative number of CFU-M that is higher than the relative number of CFU-G is associated with the presence and/or the course and/or the severity and/or the prediction of neurodegenerative diseases.

6. The method as claimed in any of claims 1 to 4, **characterized in that** in step c) a certain relative number of CFU-M is associated with the presence and/or the course and/or the severity and/or the prediction of neurodegenerative diseases.

7. The method as claimed in claim 6, **characterized in that** a number of CFU-M colonies of at least approx. 25% is associated with the presence and/or course and/or the severity and/or the prediction of neurodegenerative diseases.

8. The method as claimed in any of claims 1 to 7, **characterized in that** the medium used in step b) for cultivation contains methylcellulose.

9. The method as claimed in any of claims 1 to 8, **characterized in that** the white blood cells are isolated from the blood sample by density-gradient centrifugation or by purification by means of specific antibodies.

10. The method as claimed in any of claims 1 to 9, **characterized in that** the white blood cells isolated are cultured for a period of at least 10 days, preferably 14 days.

11. Use of a CFC-assay comprising colony-forming of hematopoietic precursor cells in a sample for diagnosing and/or predicting the development of neurodegenerative diseases.

## Revendications

1. Procédé de diagnostic et/ou de pronostic du développement de maladies neurodégénératives, le procédé comprenant les étapes suivantes :
a) l'isolement de globules blancs à partir d'un échantillon de sang d'une personne à étudier ;
b) l'enrichissement et/ou la culture des globules blancs isolés à l'étape a) pour la formation de différentes unités formant colonie (UFC), des UFC-M et d'autres UFC se formant ; et
c) la détermination et l'évaluation du nombre relatif d'UFC-M formées à l'étape b) par rapport aux autres UFC formées.

2. Procédé selon la revendication 1, **caractérisé en ce que** l'étape b) est réalisée dans un milieu pour la formation de différentes unités formant colonie (UFC).

3. Procédé selon la revendication 1 ou 2, **caractérisé en ce qu'**à l'étape c), le nombre relatif d'au moins deux des UFC suivantes formées à l'étape b) est déterminé et comparé, à savoir les UFC-G (UFC-granulocyte) et les UFC-M (UFC-macrophage).

4. Procédé selon la revendication 3, **caractérisé en ce que** le nombre relatif d'UFC-GM (UFC-granulocyte/macrophage) est en outre déterminé.

5. Procédé selon l'une quelconque des revendications 1 à 4, **caractérisé en ce que** la détermination d'un nombre relatif d'UFC-M qui est supérieur au nombre relatif d'UFC-G est associée à la présence et/ou à l'évolution et/ou à l'intensité et/ou au pronostic de maladies neurodégénératives.

6. Procédé selon l'une quelconque des revendications 1 à 4, **caractérisé en ce qu'**à l'étape c), un nombre relatif déterminé d'UFC-M est associé à la présence et/ou à l'évolution et/ou à l'intensité et/ou au pronostic de maladies neurodégénératives.

7. Procédé selon la revendication 6, **caractérisé en ce qu'**un nombre de colonies UFC-M d'au moins environ 25 % est associé à la présence et/ou à l'évolution et/ou à l'intensité et/ou au pronostic de maladies neurodégénératives.

8. Procédé selon l'une quelconque des revendications 1 à 7, **caractérisé en ce que** le milieu utilisé à l'étape b) pour la culture contient de la méthylcellulose.

9. Procédé selon l'une quelconque des revendications 1 à 8, **caractérisé en ce que** l'isolement de globules blancs à partir de l'échantillon de sang a lieu par une centrifugation en gradient de densité ou une purification au moyen d'anticorps spécifiques.

10. Procédé selon l'une quelconque des revendications 1 à 9, **caractérisé en ce que** la culture des globules blancs isolés a lieu pendant une période d'au moins 10 jours, de préférence de 14 jours.

11. Utilisation d'un essai CFC avec des cellules précurseurs hématopoïétiques de la formation de colonies dans un échantillon pour le diagnostic et/ou le pronostic du développement de maladies neurodégénératives.
